# EUROPEAN PATENT APPLICATION

(11) **EP 3 175 828 A1**
(43) Date of publication of application: **07.06.2017**
(21) Application number: 15197948.1
(22) Date of filing: 04.12.2015
(51) Int. Cl.: A61F 5/41, A61H 19/00, A61F 6/04

(54) **SEXUAL ENHANCEMENT DEVICE**

(71) Applicant: Crus Ricardez, Gameli Eduardo, North Las Vegas, NV 89032 (US)
(72) Inventor: Crus Ricardez, Gameli Eduardo, North Las Vegas, NV 89032 (US)
(74) Representative: Romano, Giuseppe

(57) **Abstract**

A sexual enhancement device is provided including an inner condom for placement on the penis of a user; an outer condom connected to the inner condom, the outer condom preferably having a second size greater than the first size of the inner condom; and a device pad between the outer condom and the inner condom. The outer condom is unrolled or extended over the device pad after the device pad is placed over the inner condom. The sexual enhancement device enhances the sexual performance of a male by thickening the penis, resulting in enhanced pleasure for both male and female participants during sexual intercourse.

## Description

### Cross Reference to Related Application

This application claims the benefit of co-pending United States Provisional Patent Application Serial No. 62/081,991, filed November 19, 2014, which is incorporated herein in its entirety.

### Field of the Invention

The present invention relates generally to sexual enhancement devices, and more particularly, to a sexual enhancement device which is simple and inexpensive in design and effectively enhances the sexual performance of a male by thickening the penis, resulting in enhanced pleasure for both male and female participants during sexual intercourse.

### Background of the Invention

Reduced sexual performance affects an estimated 15 million to 30 million men in the United States. Reduced sexual performance may be a major source of psychological stress and frustration in relationships. Conventional methods for enhancing sexual performance include reducing the quantity of drugs which may adversely affect achieving and maintaining an erection as well as psychotherapy, drug therapy such as medications and testosterone, mechanical devices such as vacuum devices, and surgical treatment.

While they may be effective, oral medicines may have negative side effects and delayed time of onset. The results of studies which test the effectiveness of some performance-enhancing drugs are inconsistent. Furthermore, oral medications may conflict with drugs which the user may be taking for other conditions. In turn, oral testosterone may reduce erectile dysfunction in some men with low levels of natural testosterone but may potentially cause liver damage.

Surgical approaches to enhancing sexual performance may include stiffening the penis by implantation of a penile prosthesis. However, these types of devices may have various drawbacks and may have a tendency to fail due to improper implementation.

Other medical approaches to enhancing sexual performance may include injection of a medication into the corpus cavernosum of the penis.

Other methods may restrict venous drainage from the corpora cavernosum of the penis by placement of a ring around the base of the penis to increase the turgor pressure in the corpora cavernosum.

Although many effective treatment modalities for enhancing sexual performance have been formulated, many of these solutions may be complicated and expensive.

Other techniques for enhancing male sexual performance may include the use of exteriorly-placed devices to stiffen the penis. These devices may include tubular sheaths which reinforce the penis. However, since these devices are largely rigid, they typically must be individually selected from among devices having different sizes. Moreover, the devices may be incapable of adapting to the volume of the penis and may potentially compromise circulation in the penis.

Condoms are commonly used to both practice contraception and to reduce the spread of sexually transmitted diseases (STDs). Some condoms are fabricated with features which enhance sexual pleasure. For example, ribbed condoms include ribs which are fabricated in the wall along the length of the condom to provide additional stimulus to the female. Additionally, condoms have been made of different materials such as lambskin to increase sensitivity of the male. Generally, however, most condom enhancements provide only modest improvements in stimulation to one or both participants.

Accordingly, there is an established need for a sexual enhancement device which is simple and inexpensive in design and effectively enhances the sexual performance of a male, resulting in enhanced pleasure for both male and female participants during sexual intercourse.

### Summary of the Invention

The present invention is directed to a sexual enhancement device which is simple and inexpensive in design and effectively enhances the sexual performance of a male by thickening the penis. The sexual enhancement device includes an inner sheath or condom which is pulled or unrolled over the erect penis of a user, a device pad which is placed in a folded position around the inner condom, and an outer sheath or condom which is connected to the inner condom and pulled or unrolled over the device pad. The sexual enhancement device imparts thickness and texture to the penis of the user to increase sexual satisfaction of the male and female participants during sexual intercourse. For example, the sexual enhancement device may assist couples in enjoying a more satisfying sexual intercourse in the event of the female participant suffering from stretching damage caused by having given vaginal birth.

In a first aspect, the present disclosure is generally directed to a male sexual enhancement device including a first inner condom having a generally cylindrical inner condom wall and terminating at a tip. A second outer condom has a generally cylindrical outer condom wall and terminates at a tip wherein the second outer condom tip is connected to the tip of the first inner condom. The male sexual enhancement device further includes a device pad, and is arrangeable to adopt a functional position in which the second outer condom is sleeved over the first inner condom, and the device pad is interposed between the inner condom wall and the outer condom wall.

In a second aspect, the diameter of the second outer condom can be greater than a diameter of the first inner condom.

In another aspect, the device pad can extend from the tips of the condom to a base of the condoms when the male sexual enhancement device is arranged in the functional position.

In yet another aspect, the device pad can include a first pad section, a second pad section, and a pad connector connecting the first pad section to the second pad section.

In a still further aspect, the pad connector can be fabricated of a resilient material and can connect a tip of the first pad section to a tip of the second pad section.

In another aspect, the male sexual enhancement device can further include a condom junction connecting the tip of the first inner condom to the tip of the second outer condom.

In another aspect, the pad connector can define a notch, wherein the notch engages the condom junction when the male sexual enhancement device is arranged in the functional position.

In a still further aspect, each of the first and second pad sections can include a respective pad section wall, wherein the respective pad section walls can be filled with a soft, resilient pad filler material.

In yet another aspect, the pad filler material can include a liquid gel.

In another aspect, the first pad section and the second pad section can be integrally formed of a uniform resilient material.

In another implementation, a male sexual enhancement device includes a first inner condom having an inner base ring, a generally cylindrical inner condom wall extending therefrom and terminating at a tip. A second outer condom has an outer base ring, a generally cylindrical outer condom wall extending therefrom and terminating at a tip connected to the tip of the first inner condom. The second outer condom has a diameter greater than the first inner condom. A condom junction connects the tip of the first inner condom to the tip of the second outer condom. The male sexual enhancement device further includes a device pad. The male sexual enhancement device is arrangeable to adopt a functional position in which the second outer condom is sleeved over the first inner condom, and the device pad is interposed between the inner condom wall and the outer condom wall extending substantially from the outer base rings to the condom junction.

In a second aspect, the device pad can include a first pad section, a second pad section, and a pad connector connecting the first pad section to the second pad section.

In another aspect, the pad connector can be fabricated of a resilient material and can connect a tip of the first pad section to a tip of the second pad section.

In still another aspect, each of the first and second pad sections can include a respective pad section wall, wherein the respective pad section walls can be filled with a soft, resilient pad filler material.

In yet another aspect, the pad filler material can include a liquid gel.

In a still further aspect, the first pad section and the second pad section can be integrally formed of a uniform resilient material.

In yet another implementation, a male sexual enhancement device includes a first inner condom having an inner base ring, a generally cylindrical inner condom wall extending therefrom and terminating at a tip. A second outer condom has an outer base ring, a generally cylindrical outer condom wall extending therefrom and terminating at a tip connected to the first inner condom tip. The second outer condom has a diameter greater than the first inner condom. A condom junction connects the tip of the first inner condom to the tip of the second outer condom. The male sexual enhancement device further includes a device pad comprising a first pad section, a second pad section, and a pad connector connecting the first pad section to the second pad section. The male sexual enhancement device is arrangeable to adopt a functional position in which the second outer condom is sleeved over the first inner condom, the device pad is interposed between the inner condom wall and the outer condom wall extending substantially from the outer base rings to the condom junction, and the pad connector engages the condom junction.

In another aspect, the pad connector can define a pad connector notch therein. The pad connector notch can receive the condom junction when the male sexual enhancement device is arranged in the functional position.

In a still further aspect, each of the first and the second pad sections can include a respective pad section wall, wherein the respective pad section walls can be filled with a soft, resilient pad filler material.

In yet another aspect, the first pad section and the second pad section can be integrally formed of a uniform resilient material.

These and other objects, features, and advantages of the present invention will become more readily apparent from the attached drawings and the detailed description of the preferred embodiments, which follow.

### Brief Description of the Drawings

The preferred embodiments of the invention will hereinafter be described in conjunction with the appended drawings provided to illustrate and not to limit the invention, where like designations denote like elements, and in which:
FIG. 1 presents a side elevation view of attached inner and outer condoms of a sexual enhancement device in accordance with an exemplary embodiment of the invention;
FIG. 2 presents an isometric perspective view of the attached inner and outer condoms of FIG. 1, more particularly illustrating placement of the inner condom on the penis of a user preparatory to typical application of the device;
FIG. 3 presents an isometric perspective view of the attached inner and outer condoms of FIG. 1 with the inner condom placed on the penis of the user preparatory to typical application of the sexual enhancement device;
FIG. 4 presents a top plan view of a device pad of the sexual enhancement device according to an exemplary embodiment of the invention, the device pad shown unfolded;
FIG. 5 presents an isometric perspective view of the device pad of FIG. 4 in a folded configuration and being placed onto the attached inner and outer condoms of FIG. 1 , more particularly over the inner condom prior to deployment of the outer condom over the device pad;
FIG. 6 presents an isometric perspective view of the assembled sexual enhancement device with the outer condom deployed over the folded device pad preparatory to use of the device;
FIG. 7 presents a side elevation view of the sexual enhancement device assembled on the penis of the user preparatory to use of the device; and
FIG. 8 presents a longitudinal cross-sectional side elevation view of the sexual enhancement device, taken along section lines 8-8 in FIG. 7, the penis having been omitted.

Like reference numerals refer to like parts throughout the several views of the drawings.

### Detailed Description

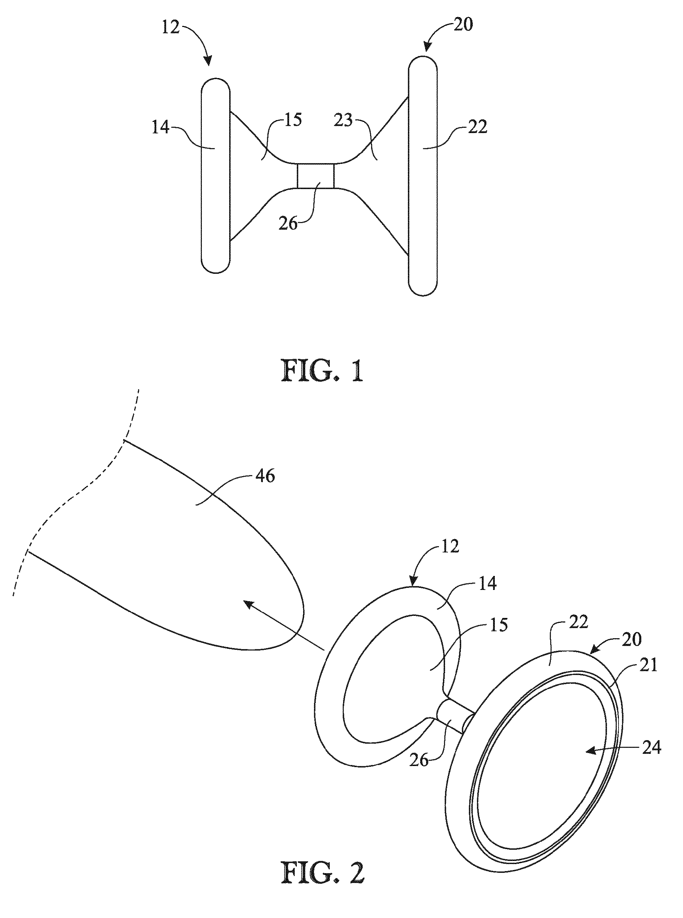

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Shown throughout the figures, the present invention is directed toward a sexual enhancement device which is simple and inexpensive in design and effectively enhances the sexual performance of a male by thickening the penis, resulting in enhanced pleasure for both male and female participants during sexual intercourse.

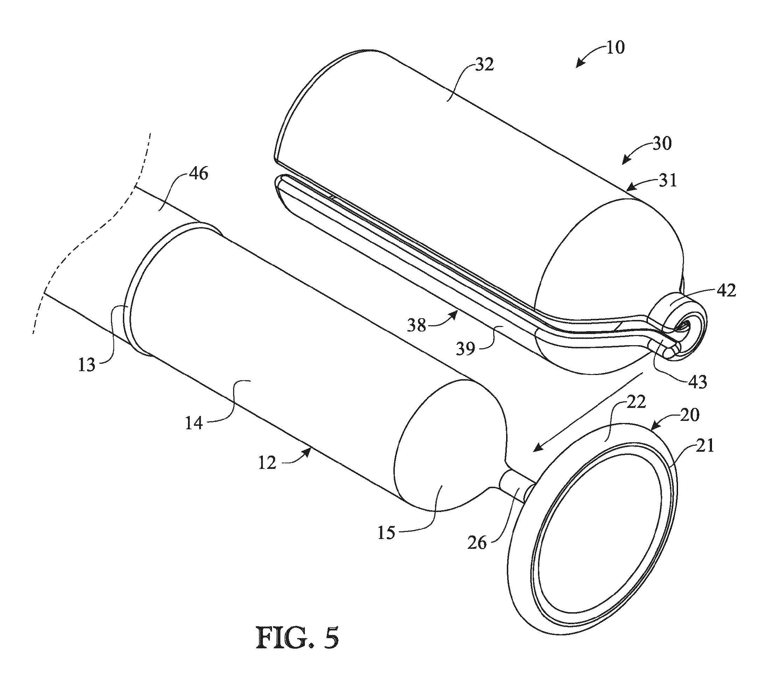

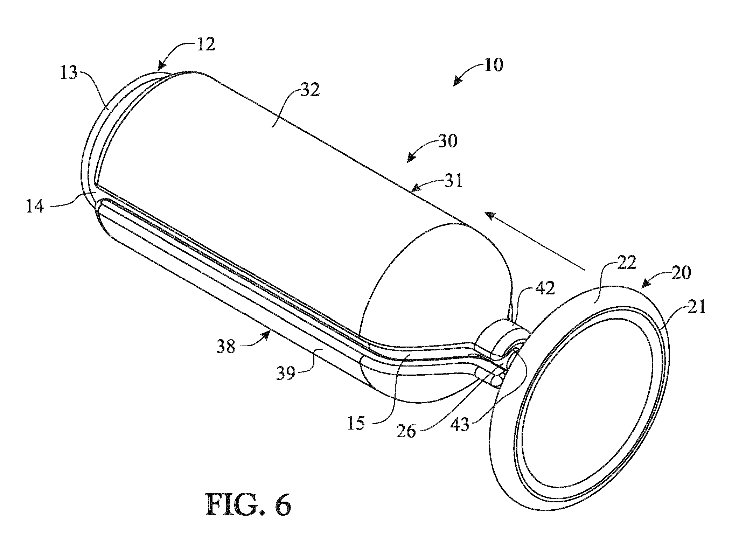

Referring initially to FIGS. 5 and 6, a sexual enhancement device 10 is illustrated in accordance with an exemplary embodiment of the present invention, assembled on the penis 46 of a male user in FIG. 5. The sexual enhancement device 10 includes a generally elastic inner sheath or condom 12 which is placed over the penis 46 of the user. A generally elastic outer sheath or condom 20 is attached to the inner condom 12. The outer condom 20 may have a size which is larger than that of the inner condom 12. A device pad 30 is disposed between the inner condom 12 and the outer condom 20. The device pad 30 imparts thickness to the sexual enhancement device 10 and enlarges the width of the user's penis 46, enhancing the sexual pleasure of both male and female participants of sexual intercourse.

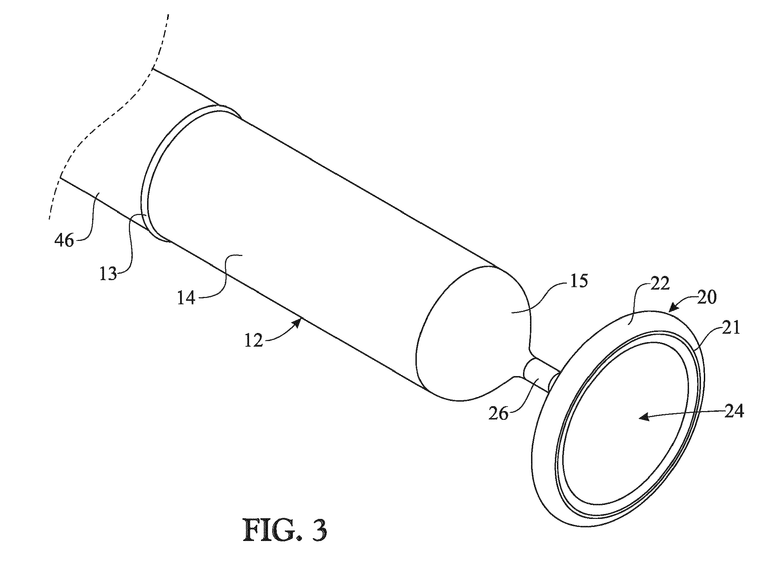

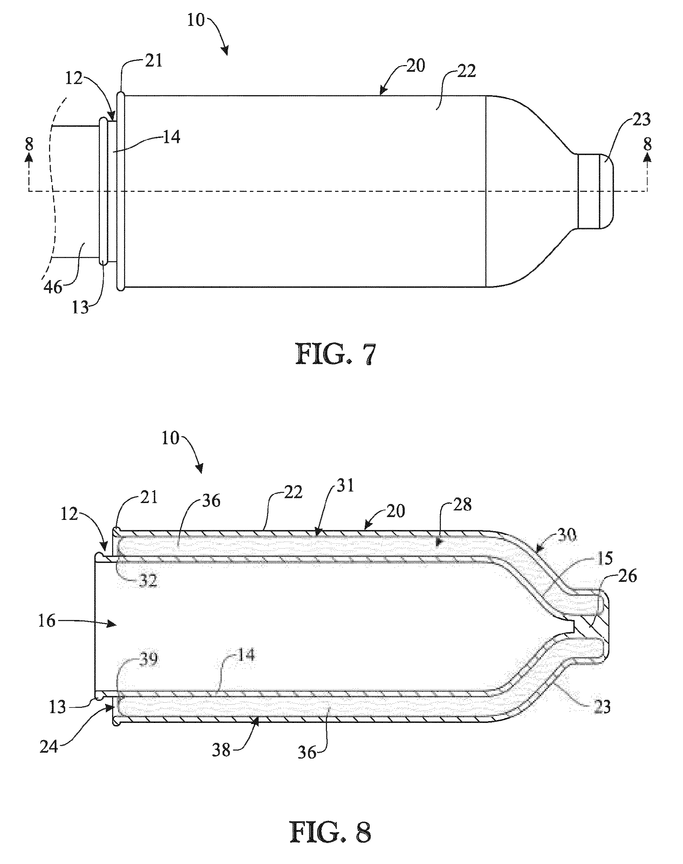

Referring next to FIGS. 1 through 4, the inner condom 12 of the sexual enhancement device 10 may have a conventional condom design known by those skilled in the art. The inner condom 12 may include an inner condom base ring 13, as shown in FIG. 3. An elongated, generally cylindrical inner condom wall 14 may extend from the inner condom base ring 13. A tapered inner condom tip 15 may extend from the inner condom wall 14. The inner condom 12 has an inner condom interior 16 (FIG. 8).

The outer condom 20 of the sexual enhancement device 10 may have a design which is the same as or similar to that of the inner condom 12. Accordingly, the outer condom 20 may include an outer condom base ring 21. An elongated, generally cylindrical outer condom wall 22 may extend from the outer condom base ring 21. A tapered outer condom tip 23 may extend from the outer condom wall 22. The outer condom 20 has an outer condom interior 24 (FIG. 8). As illustrated in FIG. 1, a condom junction 26 may connect the outer condom tip 23 of the outer condom 20 to the inner condom tip 15 of the inner condom 12. In some embodiments, the outer condom tip 23 and the inner condom tip 15 may be fabricated in one piece with the condom junction 26 using molding and/or other fabrication techniques known by those skilled in the art. In other embodiments, the outer condom tip 23 and the inner condom tip 15 may be fabricated separately and then attached or fused to each other via the condom junction 26 using any of a variety of suitable attachment techniques known by those skilled in the art. The inner condom 12 and the outer condom 20 may be fabricated of latex, polyurethane, polyisoprene, lamb intestine or other materials which are known to be suitable materials for condom fabrication. Moreover, the inner condom 12, the outer condom 20 and/or the device pad 30 may be fabricated with ribbing or other textured surface characteristics to enhance stimulation during intercourse.

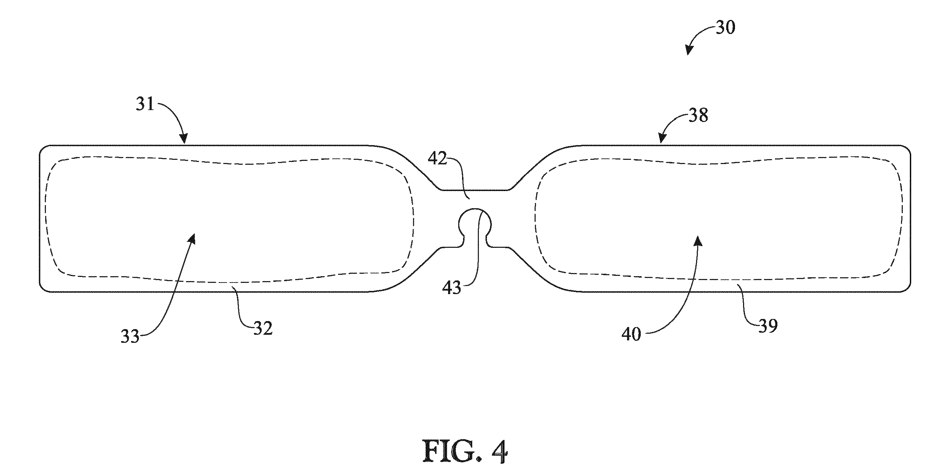

As illustrated in FIG. 4, the device pad 30 of the sexual enhancement device 10 may include a first pad section 31 and a second pad section 38. A pad connector 42 may connect the second pad section 38 to the first pad section 31. The pad connector 42 may have a pad connector notch 43. The pad connector 42 of the present embodiment is narrower than the first and second pad sections 31, 38, in a way of an intermediate connecting neck. The first pad section 31 may have a first pad section wall 32. A first condom cavity 33 may be formed within the first pad section wall 32. Similarly, the second pad section 38 may have a second pad section wall 39. A second condom cavity 40 may be formed within the second pad section wall 39. The first pad section wall 32, the second pad section wall 39 and the pad connector 42 of the device pad 30 may be fabricated of a flexible material such as silicone or latex, for example and without limitation.

As illustrated in FIG. 8, in some embodiments, a soft, resilient pad filler material 36 may fill the first pad section wall 32 of the first pad section 31 and the second pad section wall 39 of the second pad section 38 of the device pad 30. The pad filler material 36 may include a liquid such as gel, for example and without limitation. In other embodiments, the first pad section 31 and/or second pad section 38 can be integrally formed of a single material.

In various embodiments, the inner condom 12, the outer condom 20 and/or the device pad 30 may be fabricated in different textures, colors, fragrances and/or flavors. The inner condom 12 and the outer condom 20 may be rolled and packaged in a single package. The device pad 30 may be washable and reusable.

Referring next to FIGS. 2, 3 and 5 through 8, in typical application, the sexual enhancement device 10 is assembled on the penis 46 of the user by initially unrolling and pulling the inner condom 12 over the erect penis 46, as illustrated in FIGS. 2 and 3, by fitting the inner condom 12 on the erect penis 46 and unrolling the inner condom wall 14 onto the penis. Next, as illustrated in FIGS. 5 and 6, the device pad 30 may be folded around the deployed inner condom 12, so that the first condom cavity 33 (FIG. 4) in the first pad section 31 receives a first side of the penis 46, the pad connector 42 is folded at the pad connector notch 43, and the second condom cavity 40 in the second pad section 38 receives a second side of the penis 46. The pad connector notch 43 on the pad connector 42 of the device pad 30 may receive the condom junction 26 between the inner condom 12 and the outer condom 20 to secure the device pad 30 in place. The outer condom 20 is then unrolled and pulled over the device pad 30 as the outer condom tip 23 of the outer condom 20 remains attached to the inner condom tip 15 of the inner condom 12, as illustrated in FIGS. 7 and 8. Accordingly, as illustrated in FIG. 8, the device pad 30 is sandwiched in a condom space 28 between the inner condom 12 and the outer condom 20.

Throughout sexual intercourse, the sexual enhancement device 10 increases the thickness of the penis 46, enhancing the sexual pleasure of both the male and female participants. During ejaculation, semen may be collected in the inner condom interior 16 of the inner condom 12. After intercourse, the inner condom 12 may be removed from the penis 46. The outer condom 20 may be rolled from the device pad 30, which may be detached from the condom junction 26 at the pad connector notch 43. The inner condom 12 and the attached outer condom 20 may be discarded. In some applications, the device pad 30 may be washed and re-used in a similar manner.

Since many modifications, variations, and changes in detail can be made to the described preferred embodiments of the invention, it is intended that all matters in the foregoing description and shown in the accompanying drawings be interpreted as illustrative and not in a limiting sense. Thus, the scope of the invention should be determined by the appended claims and their legal equivalents.

## Claims

1. A male sexual enhancement device comprising:
a first inner condom having a generally cylindrical inner condom wall and terminating at a tip;
a second outer condom having a generally cylindrical outer condom wall and terminating at a tip connected to said tip of said first inner condom; and
a device pad; wherein
said male sexual enhancement device is arrangeable to adopt a functional position in which said second outer condom is sleeved over said first inner condom, and said device pad is interposed between said inner condom wall and said outer condom wall.

2. The male sexual enhancement device according to claim 1, wherein a diameter of said second outer condom is greater than a diameter of said first inner condom.

3. The male sexual enhancement device according to claim 1, wherein said device pad extends from said tips of said condoms to a base of said condoms when said male sexual enhancement device is arranged in said functional position.

4. The male sexual enhancement device according to claim 1, wherein said device pad comprises a first pad section, a second pad section, and a pad connector connecting said first pad section to said second pad section.

5. The male sexual enhancement device according to claim 4, wherein said pad connector is fabricated of a resilient material and connects a tip of said first pad section to a tip of said second pad section.

6. The male sexual enhancement device according to claim 5, further including a condom junction connecting said tip of said first inner condom to said tip of said second outer condom.

7. The male sexual enhancement device according to claim 6, wherein said pad connector defines a notch therein, said notch engaging said condom junction when said male sexual enhancement device is arranged in said functional position.

8. The male sexual enhancement device according to claim 4, wherein each said first and said second pad section comprises a respective pad section wall, and wherein said respective pad section walls are filled with a soft, resilient pad filler material.

9. The male sexual enhancement device according to claim 8, wherein said pad filler material comprises a liquid gel.

10. The male sexual enhancement device according to claim 4, wherein said first pad section and said second pad section are integrally formed of a uniform resilient material.
